# EUROPÄISCHE PATENTSCHRIFT

(11) **EP 3 962 574 B1**
(45) Veröffentlichungstag und Bekanntmachung des Hinweises auf die Patenterteilung: **12.08.2026**
(21) Anmeldenummer: 20725436.8
(22) Anmeldetag: 27.04.2020
(51) Int. Cl.: A61M 39/02

(54) **INTRAKORPORALES FÜHRUNGSBAUTEIL**
INTRACORPOREAL GUIDING COMPONENT
ÉLÉMENT DE GUIDAGE INTRACORPOREL

(30) Priorität: 29.04.2019 DE 102019110982
(43) Veröffentlichungstag der Anmeldung: 09.03.2022
(73) Patentinhaber: Corlife OHG, 30625 Hannover (DE)
(72) Erfinder: HAVERICH, Axel, 30177 Hannover (DE); MEYER-KOBBE, Clemens, 31157 Sarstedt (DE); HARDER, Michael, 31141 Hildesheim (DE)
(74) Vertreter: Kröncke, Rolf
(86) Internationale Anmeldenummer: PCT/EP2020/061625
(87) Internationale Veröffentlichungsnummer: WO 2020/221695

(56) Entgegenhaltungen:
- DE-T2- 69 738 378
- US-A- 4 781 694
- US-A- 5 799 661
- US-A1- 2012 130 193
- US-A1- 2013 253 263
- US-B2- 10 105 537

## Beschreibung

Die Erfindung betrifft ein intrakorporales Führungsbauteil zum Führen von Leitungen medizinischer Vorrichtungen innerhalb eines Lebewesens. Hierbei weist das Führungsbauteil einen Grundkörper mit einem Leitungskanal zur Aufnahme und Führung der Leitung auf.

Derartige Führungsbauteile dienen der Führung, Halterung und Fixierung von in einem Körper eines Lebewesens befindlichen Kabeln, Schläuchen, Röhrchen oder anderen Leitungen von medizinischen Produkten. Beispielsweise benötigen einige implantierte medizinische Vorrichtungen eine dauerhafte Energieversorgung, die in der Regel außerhalb des Körpers bereitgestellt und konstant am Körper getragen wird.

Als konkretes Beispiel können hier implantierte Kunstherzsysteme genannt werden. Die auch Driveline genannte Verbindung zwischen dem künstlichen Herz und einer externen Steuerung/Energieversorgung wird ausgehend von der Steuerung durch die Haut in den Körper des Lebewesens geleitet. Die Driveline beinhaltet in der Regel eine Energieversorgungsleitung und eine Steuer- bzw. Datenleitung zum Austausch von beispielsweise Sensor- und Messdaten oder Steuerbefehlen.

Das Führungsbauteil ist für transkutan geführte Leitungen vorgesehen. Solche Leitungen werden durch die Haut des Lebewesens hindurch geführt. Bei einer solchen Anwendung ist vorgesehen, dass die Austrittsstelle der Leitung von Haut ummantelt wird, damit diese an der Leitung, bevorzugt auch an dem Führungsbauteil anwachsen kann und eine Infektionsbarriere bildet.

Hersteller wählen in der Regel Leitungsoberflächen, die ein Anwachsen an der Haut begünstigen und so einen dichten Abschluss bilden. Hierzu darf an der Leitung jedoch keine Zuglast anliegen, da durch Bewegungen der Leitung ein Anwachsen verhindert wird. Die Leitung kann auf der Haut fixiert werden, beispielsweise mit Kunststoffflügeln, die an einem Pflaster verankert werden. Grundsätzlich ist zu beachten, dass insbesondere die transmuskuläre und/oder subkutane Passage der Leitung infektionsanfällig ist.

Die US 10,105,537 B2 offenbart eine Kabelhalterung für Schrittmacherkabel. Die Halterung wird in einem interkostalen Bereich, also zwischen zwei Rippen eines Lebewesens in oder an einem Muskelgewebe fixiert, beispielsweise angeschraubt oder angenäht. Die Halterung soll eine unerwünschte Lageänderung des Schrittmacherkabels verhindern und es gegenüber dem Muskel, durch den das Kabel geführt ist, elektrisch isolieren.

Die US 2006/0025826 A1 zeigt einen subkutan implantierbaren Kardioverter-Defibrillator mit einer teleskopierbaren Leitung. Die Vorrichtung hat ein Gehäuse mit einem Führungskanal, in dem die Leitung verschieblich angeordnet ist, um so einen Längenausgleich und eine freiere Positionierung des Gehäuses zu ermöglichen. Das Gehäuse kann zur Erhöhung des Tragekomforts interkostal parallel zu und zwischen den Rippen eines Patienten angeordnet werden. Hierzu kann das Gehäuse länglich und gekrümmt ausgeführt sein.

Der US 2018/0272122 A1 ist ein implantierbares medizinisches Gerät zu entnehmen, das an interkostalem Muskelgewebe befestigbar ist. Hierzu weist das Gerät eine oder mehrere längliche Ankerstrukturen auf, die in das Muskelgewebe eingeführt werden und das Gerät dadurch an seiner vorbestimmten interkostalen Position verankern. Das Gerät kann eine konvexe Form aufweisen, um bündig an dem Muskelgewebe angeordnet werden zu können.

In der US 2004/215303 A1 sind Kontakte für einen elektrischen Anschluss biomedizinischer implantierbarer Leitungen beschrieben. Hierbei handelt es sich um biokompatible elektrisch leitende Kontaktanschlüsse, die den anzuschließenden Leiter beispielsweise ringförmig umgeben.

Die US 2011/009933 A1 betrifft ein implantierbares elektrisches Stimulationssystem mit einem implantierbaren Gerät, das einen Führungskanal für ein oder mehrere Elektrodenleitungen aufweist.

In der US 2011/004286 A1 wird ein implantierbares Kardiogerät mit Leitung vorgeschlagen, bei dem das beispielsweise als Pulsgenerator oder Defibrillator ausgeführte Kardiogerät im Brustkorb oder der Bauchdecke eines Patienten angeordnet und die Leitung zum Herzen geführt wird. Zur Versteifung und Zugentlastung der Leitung ist abschnittsweise ein steifer Leitungskanal mit beispielsweise vier Öffnungen für die Leiterdrähte vorgesehen.

Vor diesem Hintergrund liegt der Erfindung die Aufgabe zugrunde, ein verbessertes Führungsbauteil bereitzustellen, das einfacher und gewebeschonender in dem Körper eines Lebewesens fixierbar ist. Das Führungsbauteil soll auch zur Führung von transkutan verlaufenden Leitungen geeignet sein.

Die Aufgabe wird durch ein gattungsgemäßes Führungsbauteil gelöst, bei dem der Grundkörper des Führungsbauteils an seinem Umfang eine oder mehrere Anlageflächen aufweist, die zur formschlüssigen und/oder kraftschlüssigen Anlage an einer Knochenstruktur des Lebewesens ausgebildet sind.

Unter einer formschlüssigen Anlage wird eine Verbindung in Form eines Ineinandergreifens von Führungsbauteil und Knochenstruktur verstanden, bei der sich das Führungsbauteil und die Knochenstruktur aufgrund ihrer Form nicht voneinander lösen. Hierbei sind sich das Führungsbauteil und die Knochenstruktur gewissermaßen "im Weg", sodass sie sich gegeneinander abstützen.

Bei einer kraftschlüssigen Anlage wird eine gegenseitige Verschiebung zwischen Führungsbauteil und Knochenstruktur aufgrund der zwischen beiden Komponenten wirkenden Haftreibung verhindert. Solange die Haftreibung nicht durch eine äußere Krafteinwirkung überwunden wird, haften das Bauteil und die Knochenstruktur reibschlüssig aneinander.

Der Grundkörper kann somit einerseits durch seine Form, die beispielsweise abschnittsweise eine Negativkontur oder einen Hinterschnitt einer Knochenstruktur abbildet, und andererseits durch seine Oberfläche, die beispielsweise einen hohen Haftreibungskoeffizienten aufweist, an der Knochenstruktur gehalten sein. Selbstverständlich ist auch eine Kombination aus einer form- und einer kraftschlüssigen Anlage denkbar.

Die Formschlüssigkeit kann beispielsweise durch eine zumindest abschnittsweise gebogene Form des Führungsbauteils in Zusammenwirkung mit einer Rippenbiegung bereitgestellt werden. Entsprechende Einkerbungen der kranialen und kaudalen Flächen des Führungsbauteils können hierbei die Formschlüssigkeit erhöhen.

Erfindungsgemäß ist eine Anlagefläche, die zumindest einen Abschnitt der Oberfläche des Grundkörpers einnimmt, zur form- und/oder kraftschlüssigen Anlage an der Knochenstruktur ausgebildet. Die Anlagefläche kann auch umlaufend an einem Umfang des Grundkörpers vorgesehen oder in mehrere einzelne, räumlich voneinander beabstandete Anlageflächen aufgeteilt sein. Es muss also nicht der gesamte Grundkörper zur Anlage an der Knochenstruktur ausgebildet sein.

Unter einer Knochenstruktur werden druck- und zugfeste Organe des Lebewesens verstanden, die Teil seines Skeletts sind. Hierbei kann es sich um einen einzelnen Knochen oder auch um mehrere Knochen oder Knochenverbindungen wie Gelenke handeln. Bevorzugt handelt es sich bei der Knochenstruktur um eine Rippenstruktur, beispielsweise eines Brustkorbs des Lebewesens, wobei die Rippenstruktur durch eine einzelne Rippe oder mehrere, insbesondere zwei benachbarte Rippen gebildet sein kann.

Als Leitungen kommen grundsätzlich auch starre, aber insbesondere flexible oder zumindest abschnittsweise flexible Leitungen in Betracht, also biegsame und/oder elastische Verbindungskanäle, die geeignet sind, ein Medium zu führen oder die als Energieversorgungs- und/oder Datenleitung ausgelegt sind. Diese können zur Energieversorgung oder Datenleitung beispielsweise elektrische Leiter wie metallische Drähte, aber auch optische Leiter wie beispielsweise Lichtwellenleiter enthalten. Als medienführende Leitungen kommen etwa Schläuche, Röhrchen oder Katheter in Betracht, die beispielsweise Gase und Gasgemische wie Luft oder Flüssigkeiten wie etwa Körperflüssigkeiten oder medizinische Lösungen führen. Es können auch mehrere Leiter in einer diese bündelnden Leitung vorgesehen sein, beispielsweise ein Kabel oder Schlauch mit mehreren darin geführten elektrischen Leitungen wie einer Energieversorgungs- und einer Datenleitung.

In der Regel ist die Leitung mit einer medizinischen Vorrichtung verbunden, die beispielsweise als Steuerung eines Kunstherzsystems, als Dialysegerät oder als Beatmungsgerät ausgeführt sein kann. Grundsätzlich ist eine hohe Vielfalt an medizinischen Vorrichtungen denkbar, die über Leitungen mit inneren Organen eines Lebewesens verbunden sein können. Hierbei muss es sich nicht um Vorrichtungen handeln, die dauerhaft am Körper des Lebewesens getragen werden, sondern gegebenenfalls auch nur regelmäßig oder unregelmäßig an die Leitung angeschlossen werden. Die Leitung selbst ist jedoch bevorzugt dauerhaft und nicht nur temporär in dem betroffenen Lebewesen verlegt, sodass auch das Führungsbauteil für einen längerfristigen Einsatz vorgesehen ist.

Unter einem Lebewesen werden in dieser Anmeldung vorrangig menschliche oder tierische Lebewesen verstanden, für die eine medizinische Behandlung mit einer gattungsgemäßen medizinischen Vorrichtung in Frage kommt.

Das erfindungsgemäße Führungsbauteil weist einen Grundkörper auf, der die wesentliche dreidimensionale Ausdehnung und Form des Führungsbauteils definiert. Der Grundkörper kann grundsätzlich eine regelmäßige oder symmetrische Grundform wie eine Kugel-, Quader-, Zylinder-, Würfel- oder Pyramidenstumpfform aufweisen. Es sind jedoch ebenso unregelmäßige, vieleckige und asymmetrische Grundformen denkbar. In einer Ausführungsform der Erfindung hat das Führungsbauteil eine trapezförmige Grundfläche, deren schmalere Seite in einem Einbauzustand des Führungsbauteils bevorzugt dorsal orientiert sein kann. Nicht zuletzt richtet sich die Form und Ausdehnung des Grundkörpers nach den geometrischen Beschaffenheiten des intrakorporalen Bestimmungsortes, insbesondere hinsichtlich der erfindungsgemäß vorgesehenen Anlage des Grundkörpers an einer Knochenstruktur des Lebewesens.

Unabhängig von der Grundform des Grundkörpers weist dieser einen Leitungskanal zur Aufnahme und Führung der Leitung auf. Ein solcher Leitungskanal kann im einfachsten Fall als Durchgangsbohrung durch den Grundkörper ausgeführt sein, sodass ein Leitungskanal mit einem kreisrunden Führungsquerschnitt gebildet ist. Selbstverständlich sind jedoch auch Leitungskanäle mit anderen, beispielsweise rechteckigen oder halbkreisförmigen Führungsquerschnitten denkbar. Bevorzugt führt der Leitungskanal durch das Innere des Grundkörpers, um eine hohe Führungsstabilität zu gewährleisten. In anderen Ausführungsformen ist jedoch auch eine Anordnung des Leitungskanals am Umfang des Grundkörpers denkbar, beispielsweise in Form von einer geeigneten Nut oder Führungsösen, die das Lösen der Leitung von dem Grundkörper verhindern.

Es ist zu beachten, dass "ein" oder "eine" in dieser Anmeldung nicht als Zahlwort zu verstehen ist. Somit können grundsätzlich auch mehrere Leitungskanäle in einem Grundkörper bzw. einem Führungsbauteil vorgesehen sein, um mehrere unterschiedliche Leitungen halten und fixieren zu können. Insbesondere können mehrere unterschiedlich ausgebildete Leitungskanäle vorgesehen sein, die etwa unterschiedliche Durchmesser aufweisen.

Zur Herstellung des Grundkörpers kommt eine Vielzahl urformender und umformender Fertigungsverfahren in Frage. Beispielsweise kann der Grundkörper besonders einfach durch ein Gieß- oder Pressverfahren oder hochgradig individuell durch ein additives Fertigungsverfahren (zum Beispiel ein 3D-Druckverfahren) oder ein Modellierverfahren hergestellt sein. Hierbei können die Grundform ergänzende Strukturen wie beispielsweise der Leitungskanal bereits bei dem Herstellungsverfahren berücksichtigt sein, um in möglichst wenigen Herstellungsschritten ein Führungsbauteil mit den gewünschten geometrischen Anforderungen herzustellen.

Mit dem erfindungsgemäßen Führungsbauteil gelingt es erstmals, dieses schnell und einfach an der vorgesehenen Stelle im Körper des Lebewesens durch Anlage an einer oder mehreren Knochenstrukturen fixieren, beispielsweise anklemmen zu können. Somit werden keine zusätzlichen Verbindungsmittel wie insbesondere Schrauben oder chirurgische Fäden zur Anbringung des Führungsbauteils benötigt. Überdies werden Verletzungen an Gewebe, Haut und Knochen vermieden oder zumindest verringert, da das Führungsbauteil nicht zwangsläufig an diese angeschraubt oder angenäht werden muss. Somit handelt es sich um ein Führungsbauteil, das grundsätzlich minimal-invasiv implantierbar ist. Überdies ist das an der Knochenstruktur anliegende Führungsbauteil stabiler, insbesondere positions- und lagestabiler angeordnet, da es sich an der vergleichsweise festen oder lagefesten Knochenstruktur abstützt.

Das Führungsbauteil kann aufgrund seiner kraft- und/oder formschlüssigen Fixierung vergleichsweise klein dimensioniert sein, sodass im Vergleich zu Lösungen aus dem Stand der Technik nur ein kleiner Gewebeschnitt für die Implantation des Führungsbauteils erforderlich ist.

Das Führungsbauteil ist auch zur Führung von transkutan verlaufenden Leitungen geeignet, da es durch seine form- und/oder kraftschlüssige Anlage an einer Knochenstruktur direkt unter der Haut positioniert und dort sicher gehalten werden kann.

In einer besonders vorteilhaften Ausführungsform weist der Grundkörper mindestens zwei Anlageflächen auf, die zur form- und/oder kraftschlüssigen Anlage an Knochenstrukturen des Lebewesens ausgebildet sind und die einander gegenüberliegen. Somit sind die beiden Anlageflächen voneinander beabstandet, und zwar derart, dass die Anlagenflächen in unterschiedlichen Ebenen oder an unterschiedlichen Seiten des Grundkörpers ausgebildet sind. Beispielsweise können die Anlageflächen an einer Ober- und einer Unterseite und/oder an einer linken oder rechten Seite des Grundkörpers ausgebildet sein. In Bezug auf anatomische Richtungsbezeichnungen bei in den Körper des Lebewesens eingesetztem Führungsbauteil können die Anlageflächen beispielsweise auf einer kranialen und einer kaudalen Ebene und/oder auf einer dorsalen und einer ventralen Ebene des Grundkörpers angeordnet sein. Diese Ausführungsform zielt somit grundsätzlich darauf ab, dass das Führungsbauteil zwischen zwei einander gegenüberliegenden Knochenstrukturen des Lebewesens fixierbar ist. Dies begünstigt beispielsweise ein kraftschlüssiges Einklemmen zwischen den Knochenstrukturen. Hierzu kann beispielsweise der Abstand zwischen den beiden Anlageflächen etwas größer bemessen sein als der Abstand zwischen den zur Anlage vorgesehenen Knochenstrukturen, sodass eine Übermaßpassung den kraftschlüssigen Halt des Führungsbauteils zwischen den Knochenstrukturen begünstigt. Die Übermaßung kann auch durch eine ausgewölbte Ausführung einer Anlagefläche, beispielsweise der kranialen oder kaudalen Anlagefläche erzeugt werden. Grundsätzlich kann selbstverständlich auch die Form der Anlageflächen so gestaltet sein, beispielsweise als Negativkontur oder Hinterschnitt der zur Anlage vorgesehenen Knochenstrukturen, dass sich ein Formschluss des Führungsbauteils an den Knochenstrukturen ergibt. Durch die Ausführungsform mit mindestens zwei einander gegenüberliegenden Anlageflächen wird der Halt des Führungsbauteils an der vorgesehenen Stelle in dem Lebewesen verbessert. Überdies wird die Positionierung erleichtert, da die mehreren Anlageflächen mit den jeweils zugeordneten Knochenstrukturen die Positionierungsmöglichkeiten des Führungsbauteils deutlicher definieren.

In einer besonders günstigen Ausführungsform ist das Führungsbauteil als interkostales Führungsbauteil ausgebildet, dessen Grundkörper eine kraniale Anlagefläche aufweist, die zur form- und/oder kraftschlüssigen Anlage an einer kranial gelegenen Rippe des Lebewesens vorgesehen ist, und dessen Grundkörper eine kaudale Anlagefläche aufweist, die zur form- und/oder kraftschlüssigen Anlage an einer kaudal gelegenen Rippe des Lebewesens vorgesehen ist. Somit ist vorgesehen, dass das Führungsbauteil zwischen zwei benachbarten bzw. einander gegenüberliegenden Rippen positionierbar ist, von denen eine Rippe die obere bzw. kranial gelegene Rippe bildet und die andere Rippe die untere bzw. kaudal gelegene Rippe bildet.

Selbstverständlich muss das Führungsbauteil hierbei nicht über die gesamte Länge der Rippe an dieser anliegen. Grundsätzlich ist eher vorgesehen, dass das Führungsbauteil an einem Teilabschnitt der jeweiligen Rippe anliegt. Günstigerweise sind die kraniale und die kaudale Anlagefläche des Grundkörpers an die Rippenform im Bereich der Anlage des Führungsbauteils an der Rippe angepasst und hierzu beispielsweise konkav ausgeführt, um an die konvexe Form des Rippenbogens anlegbar zu sein und somit einen Formschluss zu erzeugen. Die Höhe des Führungsbauteils entspricht dem Abstand der zur Anlage vorgesehenen Rippenabschnitte voneinander. Alternativ oder zusätzlich ist eine leichte Übermaßpassung des Führungsbauteils günstig, um eine ausreichende Klemmkraft auf die anliegenden Rippenabschnitte ausüben zu können. Das Übermaß sollte jedoch nicht zu hoch gewählt werden, um kein starkes Druck- und Fremdkörpergefühl bei dem Lebewesen oder gar eine Beschädigung von Gewebe, Knochenstruktur oder Führungsbauteil zu erzeugen und um keine zu große Kraftanstrengung bei dem Einsetzen des Führungsbauteils zu erfordern. Der Vorteil der beschriebenen Ausführungsform als interkostales Führungsbauteil liegt in der besonders hohen Eignung der Rippenstruktur als Anlagepartner für die Anlageflächen des Führungsbauteils. Hierbei kann der das Führungsbauteil Implantierende relativ frei und individuell entscheiden, zwischen welchen Rippenbögen und an welcher Position das Führungsbauteil zu platzieren ist, da die Rippenstruktur vergleichsweise regelmäßig aufgebaut ist und mehrere Rippenpaare als Anlagepartner für das Führungsbauteil in Frage kommen. Diese Ausführungsform ist zudem für herzbezogene medizinische Anwendungen vorteilhaft, da die Leitung herznah in dem Brustkorb des Lebewesens mit dem Führungsbauteil gehalten werden kann. Hierdurch werden auf die Leitung wirkende Druck- und Zugkräfte herznah von dem Führungsbauteil aufgenommen und die an dem Herzen befindlichen Komponenten der medizinischen Vorrichtung mechanisch entlastet.

Diese an die Rippenstruktur des Lebewesens angepasste Ausführungsform des Führungsbauteils eignet sich überdies besonders für transkutan geführte Leitungen, da das Führungsbauteil dicht hinter dem Hautdurchtritt der Leitung an den Rippen platzierbar ist, sodass ein Anwachsen der Leitung und/oder des Führungsbauteils begünstigt wird.

Gemäß einer vorteilhaften Ausführungsform ist das Führungsbauteil mehrteilig aus mehreren Führungsbauteilkomponenten ausgebildet. Besonders bevorzugt ist das Führungsbauteil hierbei zweiteilig ausgeführt, also aus zwei Führungsbauteilkomponenten gebildet. Insbesondere ist das Führungsbauteil hierbei aus zwei Hälften zusammensetzbar. Die Teilungsebene des Führungsbauteils liegt beispielsweise in der Ebene des Leitungskanals. Somit ist an jeder der Führungsbauteilkomponenten ein Teil des Leitungskanals angeformt, sodass der Leitungskanal erst durch Zusammensetzen der Führungsbauteilkomponenten geschlossen wird. Hierdurch ist es möglich, das Führungsbauteil besonders schonend um eine bereits in dem Körper des Lebewesens verlegte Leitung herum anzuordnen, ohne die Leitung durch das geschlossene Führungsbauteil vom Leitungsende an hindurchfädeln zu müssen. Somit ergibt sich auch der Vorteil einer Nachrüstbarkeit, da das Führungsbauteil selbst bei bereits angewachsenen Leitungen noch implantierbar ist. **In** der Praxis ist es teilweise noch nicht einmal möglich, die Leitung durch das Führungsbauteil hindurchzufädeln, da die Leitung einen Stecker mit einem deutlich breiteren Querschnitt als die Leitung aufweisen kann. Das Vorsehen eines größeren Leitungskanals, durch den die Leitung mitsamt Stecker hindurchführbar wäre, würde Nachteile hinsichtlich des sicheren Haltens und Fixierens der Leitung in dem Leitungskanal mit sich bringen. Durch eine Mehrteiligkeit des Führungsbauteils, insbesondere mit einer durch den Leitungskanal verlaufenden Teilungsebene, kann der Leitungskanal hingegen optimal an den Querschnitt oder Durchmesser der Leitung angepasst werden.

Günstig ist es, wenn die Führungsbauteilkomponenten des mehrteiligen Führungsbauteils Verbindungsmittel zur Herstellung einer insbesondere lösbaren Verbindung untereinander aufweisen. Hierbei handelt es sich insbesondere um vorzugsweise direkt an die Führungsbauteilkomponenten angeformte, insbesondere einteilig mit diesen ausgebildete Verbindungsmittel, damit keine zusätzlichen, gesonderten Verbindungsmittel wie z.B. Schrauben bereitzuhalten sind. Insbesondere handelt es sich um Verbindungsmittel zur Herstellung einer Steck-, Rast- oder Clipverbindung. So kann eine Führungsbauteilkomponente eine Rastnase und eine andere Führungsbauteilkomponente eine Rastöffnung aufweisen, in der die Rastnase verrastbar ist. Durch die vorbeschriebenen Verbindungsmittel können die Führungsbauteilkomponenten besonders schnell und einfach und insbesondere werkzeuglos zusammengesteckt und auch wieder voneinander gelöst werden, beispielsweise um das Führungsbauteil auszutauschen. Die Führungsbauteilkomponenten können um die zu haltende Leitung herum positioniert und anschließend einfach durch Schließen der Rastverbindung miteinander verbunden werden, ohne dass die Leitung über ihre vollständige Länge durch den Leitungskanal der bereits zusammengesetzten Führungsbauteilkomponenten durchgeführt werden muss. Bevorzugterweise ist das mindestens eine Verbindungsmittel auf einer ventralen Ebene oder Fläche des Führungsbauteils angeordnet. Hierdurch kann bei einer Implantierung, die von der ventralen Seite des Lebewesens aus vorgenommen wird, eine bessere Zugänglichkeit und Handhabbarkeit des Verbindungsmittels gewährleistet werden.

Vorteilhafterweise weist der Grundkörper des Führungsbauteils eine um eine kranial-kaudale Mittelachse des Grundkörpers gebogene Grundform auf. Die Mittelachse verläuft somit im eingesetzten Zustand des Führungsbauteils von der kranialen in die kaudale Richtung bzw. umgekehrt. Die Biegung folgt hierbei insbesondere der Biegung einer Knochenstruktur, an der eine Anlagefläche des Führungsbauteils anliegt. Im Falle einer Rippe beispielsweise verläuft diese horizontal gesehen in einem Bogen, dem das Führungsbauteil in seiner Form folgen kann. Somit ist das Führungsbauteil durch die gebogene Grundform besser an den Verlauf der Rippe oder einer vergleichbaren Knochenstruktur angepasst, sodass der Halt des Führungsbauteils weiter verbessert wird. Hierbei ist zu beachten, dass der gebogene Verlauf der Rippe in horizontaler Betrachtungsweise nicht mit dem gebogenen Querschnitt der Rippe gleichzusetzen ist, die von dem Führungsbauteil beispielsweise durch eine konkave Kontur der Anlageflächen berücksichtigt wird.

Gemäß einer günstigen Ausführungsform verläuft der Leitungskanal zwischen einer ventralen Oberfläche des Grundkörpers und einer dorsalen Oberfläche des Grundkörpers. Somit wird die Leitung auf kürzestem Wege von einem inneren Organ in Richtung Haut des Menschen auf der ventralen oder dorsalen Seite geführt. Zudem unterstützt dieses Merkmal Ausführungsformen, bei denen das Führungsbauteil zwischen einer kranialen und einer kaudalen Knochenstruktur positioniert ist, sodass der Führungskanal an seinem Eingang oder Ausgang nicht von der Knochenstruktur blockiert oder beeinträchtigt ist.

Vorteilhaft ist es, wenn der Leitungskanal zur Aufnahme und Führung der Leitung als Schrägbohrung durch den Grundkörper ausgeführt ist. Somit verläuft der Leitungskanal im in den Körper eingesetzten Zustand des Führungsbauteils weder streng lotrecht noch horizontal. Bei einer solchen Ausführungsform sind besonders wenig bis keine Leitungsumlenkungen zu beobachten, sodass die beabsichtigte Zugentlastung maximiert wird. Überdies findet kein Verhaken und insbesondere kein Knicken der Leitung am Eingang oder Ausgang des Leitungskanals statt, sodass eine Beschädigung der Leitung während der Führung durch das Führungsbauteil verhindert wird. Auf diese Weise wird ein Kabelbruch oder Kabelknicken zuverlässig vermieden. Bei einer transkutan geführten Leitung wird zudem eine hautnahe Verlegung des extrakorporalen Abschnitts der Leitung ermöglicht. Der extrakorporale sowie auch der intrakorporale Abschnitt der Leitung wird hierbei nicht oder nur geringfügig gebogen, sodass keine oder nur geringe Kräfte auf die Austrittsstelle ausgeübt werden. Dies begünstigt ein schnelles Verheilen und Anwachsen des Gewebes an die Leitung und/oder das Führungsbauteil.

Günstigerweise weist der Leitungskanal abschnittsweise eine Querschnittsverengung auf. Alternativ oder zusätzlich ragt eine Fixierungsstruktur des Grundkörpers in den Leitungskanal hinein. Eine solche Fixierungsstruktur kann beispielsweise durch Noppen, Materialspitzen, Querstege oder vergleichbare Materialvorsprünge gebildet sein. Auf diese Weise wird die Leitung innerhalb des Leitungskanals gezielt bzw. bedarfsweise fixiert und zugentlastet. Somit werden unerwünschte Verlagerungen der Leitung reduziert oder verhindert und die Zugstabilität der Leitung vergrößert.

Bevorzugt weist der Grundkörper mindestens eine einteilig mit dem Grundkörper ausgeformte Befestigungsstruktur auf. Die Befestigungsstruktur kann insbesondere an den lateralen Flächen, also insbesondere an der ventralen oder dorsalen Oberfläche des Führungsbauteils vorgesehen sein, um die Fixierung des Führungsbauteils zu erleichtern. Die Befestigungsstruktur kann beispielsweise um den Kabelkanal herum auf einer peripheren Fläche des Kabelkanals angeordnet sein. Bei einer solchen Befestigungsstruktur kann es sich beispielsweise um eine oder mehrere Ösen handeln, sodass das Führungsbauteil bedarfsweise zusätzlich an umliegendem Gewebe, beispielsweise Muskeln, Fascien, Haut oder Fettgewebe oder auch an um- oder anliegenden Knochenstrukturen annähbar und somit fixierbar ist. Auch andere Befestigungsstrukturen wie beispielsweise Klammer- oder Federelemente sind denkbar. Insgesamt wird durch die Befestigungsstruktur der Halt des Führungsbauteils an der vorgesehenen Position im Körper des Lebewesens verbessert, wobei die Einteiligkeit der Befestigungsstruktur mit dem Grundkörper die Herstellung des Führungsbauteils vereinfacht.

In einer günstigen Ausführungsform weist der Grundkörper im Bereich des Leitungskanals einen ventralen Befestigungskranz auf. Bei einer transkutan geführten Leitung dient der Befestigungskranz der Fixierung der Haut an dem Führungsbauteil, sodass ein dichter Abschluss ermöglicht wird.

In einer vorteilhaften Ausgestaltung weist der Grundkörper mindestens eine Befestigungsöffnung auf. Durch solche Befestigungsöffnungen, bei denen es sich bevorzugt um Durchgangsbohrungen handelt, können bedarfsweise Befestigungsmittel wie beispielsweise Fäden oder Schrauben geführt werden, um den Grundkörper des Führungsbauteils bei Bedarf zusätzlich mit einer Gewebe- oder Knochenstruktur zu verbinden, beispielsweise das Führungsbauteil an eine Knochenstruktur anzuschrauben. Eine solche zusätzliche Verbindung ist etwa dann sinnvoll, wenn die erwarteten mechanischen Belastungen an der Leitung oder dem Führungsbauteil kurzzeitig die Grenzkraft der kraft- und/oder formschlüssigen Anlage des Führungsbauteils an der Knochenstruktur überschreiten können und somit der Halt des Führungsbauteils zusätzlich unterstützt werden sollte.

Vorliegend wird unter einer Befestigungsstruktur, Haltestruktur oder Fixierungsstruktur ein Materialzusatz verstanden, während eine Befestigungsöffnung mit einer Materialaussparung einhergeht.

Günstig ist es, wenn die eine oder mehreren Anlageflächen als zumindest abschnittsweise am Umfang des Grundkörpers verlaufende Nut oder Nuten ausgebildet sind. Dies entspricht grundsätzlich im Wesentlichen einer konkaven Ausführungsform der Anlageflächen, wobei die Form der betrachteten Anlagefläche einen Nutgrund mit entsprechenden Seitenwänden definiert. Eine solche Nut kann am gesamten Umfang des Grundkörpers vorgesehen sein, sodass eine freiere Orientierung des Führungsbauteils bei seiner Implantierung ermöglicht wird. Es können jedoch auch beispielsweise zwei einander gegenüberliegende Anlageflächen als Nut ausgebildet sein. Die Ausführung der Anlageflächen als Nut oder Nuten vereinfacht die Herstellung des Führungsbauteils, verbessert die Passform desselben, erleichtert das An- oder Einpressen bzw. An- oder Einklemmen des Führungsbauteils an Knochenstrukturen und erhöht die individuelle Freiheit bei der Positionierung des Führungsbauteils. Ein besonderer Vorteil der Nut oder Nuten liegt darin, dass an der Knochenstruktur befindliche Gefäße wie Blutgefäße, beispielsweise die Arteria intercostales, quetschfrei in der Nut aufgenommen werden, sodass die Blutzirkulation an der Knochenstruktur nicht durch das Führungsbauteil beeinträchtigt wird.

Bevorzugt ist am Nutgrund der Nut oder Nuten mindestens eine Haltestruktur angeordnet, insbesondere einteilig mit dem Grundkörper ausgeformt. Dies können beispielsweise angeformte Noppen oder Querstege sein. Die Noppen können beispielsweise als spitze Noppen mit einer Pyramiden- oder Kegelgrundform ausgebildet sein. Dies Haltestruktur sichert das Führungsbauteil zusätzlich gegen eine Verlagerung gegenüber der Knochenstruktur, indem die Haltestruktur beispielsweise die zwischen beiden Anlagepartnern wechselwirkende Oberfläche und somit die bestehenden Reibungskräfte erhöht.

Die Oberflächenbeschaffenheit des Führungsbauteils sollte zumindest an den Anlageflächen, bevorzugt jedoch am gesamten Führungsbauteil eine möglichst hohe Rauhigkeit aufweisen, indem beispielsweise eine mechanisch oder chemisch aufgerauhte Oberfläche bereitgestellt wird. Hiermit wird ein besseres Einwachsen des Führungsbauteils ermöglicht und die Ortsstabilität an den Rippen gefördert. Die Rauhigkeit an einzelnen Flächen, beispielsweise der kaudalen Anlagefläche, kann auch durch Spitzen wie beispielsweise kegel- oder pyramidenförmige Materialvorsprünge erhöht werden. Die Rauhigkeit erhöht den Haftreibungskoeffizienten und somit den erzielbaren Kraftschluss des Führungsbauteils mit anliegenden Strukturen.

Gemäß einer vorteilhaften Ausführungsform entspricht der Durchmesser des Leitungskanals dem Durchmesser der von dem Leitungskanal aufzunehmenden Leitung. Durch die damit einhergehende bündige Führung der Leitung in dem Leitungskanal wird unter anderem der Eintritt von Flüssigkeiten oder Gewebe in den Leitungskanal verhindert, sodass es nicht zu Gewebequetschungen oder Flüssigkeitsanstauungen kommen kann. Hierdurch wird somit auch die Verletzungs- und Entzündungsgefahr an dem Führungsbauteil reduziert. Überdies wird bereits durch die reibschlüssige Aufnahme der Leitung in dem Leitungskanal eine Lagefixierung der Leitung gegen axiale Verlagerungen erreicht.

**In** einer günstigen Ausgestaltung ist das Führungsbauteil aus einem biokompatiblen Werkstoff, insbesondere aus einem biokompatiblen Metall oder Kunststoff gefertigt. Hierdurch wird eine hohe Materialverträglichkeit bei gleichzeitig hoher Stabilität und Lebensdauer des Führungsbauteils erzielt.

Die Erfindung wird nachfolgend anhand von Ausführungsbeispielen mit den beigefügten Zeichnungen näher erläutert. Es zeigen in schematischer Weise:
- Figur 1: - eine dorsale Draufsicht eines zweiteiligen Führungsbauteils im zusammengesteckten Zustand;
- Figur 2: - eine perspektivische dorsale Ansicht des zweiteiligen Führungsbauteils im zusammengesteckten Zustand;
- Figur 3: - eine kaudale Ansicht des zweiteiligen Führungsbauteils im zusammengesteckten Zustand;
- Figur 4: - eine Seitenansicht des zweiteiligen Führungsbauteils im zusammengesteckten Zustand;
- Figur 5: - eine weitere Seitenansicht des zweiteiligen Führungsbauteils im zusammengesteckten Zustand;
- Figur 6: - eine perspektivische dorsale Ansicht des zweiteiligen Führungsbauteils mit voneinander getrennten Führungsbauteilkomponenten;
- Figur 7: - eine perspektivische ventrale Ansicht des zweiteiligen Führungsbauteils mit voneinander getrennten Führungsbauteilkomponenten.

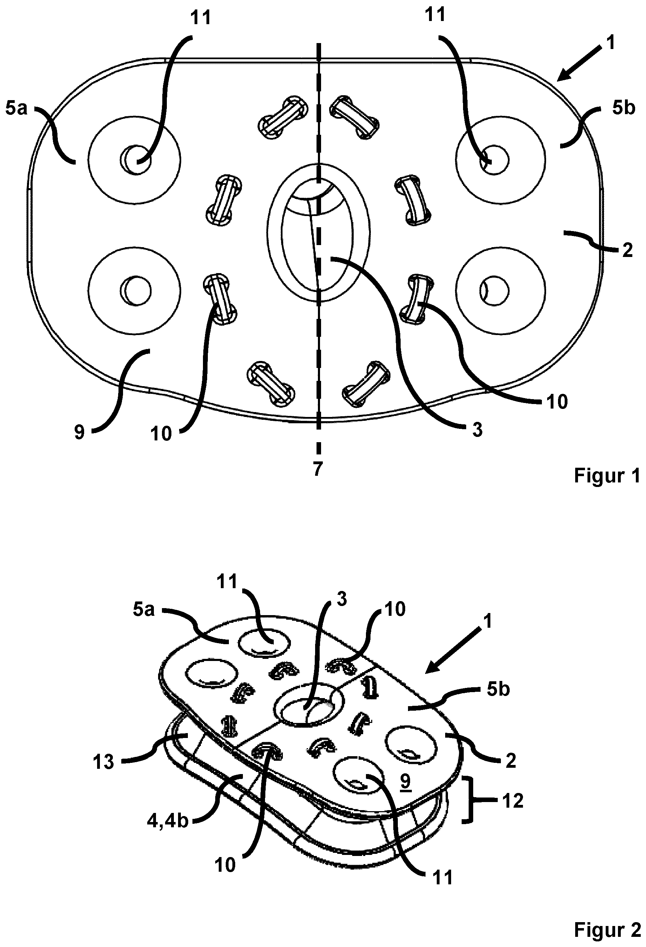

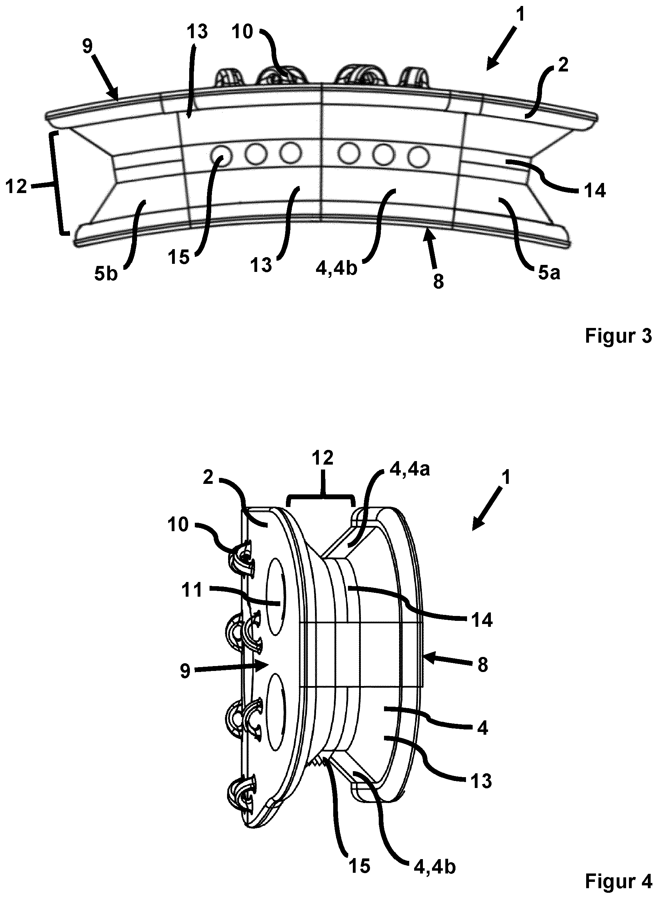

Figur 1 zeigt eine mehrteilige, nämlich zweiteilige Ausführungsform des erfindungsgemäßen Führungsbauteils 1, das aus zwei lösbar miteinander verbundenen Führungsbauteilkomponenten 5a, 5b gebildet ist. Das Führungsbauteil 1 ist dazu bestimmt, eine oder mehrere nicht gezeigte Leitungen von medizinischen Vorrichtungen in einem Körper eines Lebewesens zu führen, zu halten und zu fixieren. Hierzu weist das Führungsbauteil 1 in den weiteren Figuren gezeigte Anlageflächen 4 auf, die zur formschlüssigen und/oder kraftschlüssigen Anlage an einer Knochenstruktur des Lebewesens ausgebildet sind. Hierdurch gelingt es, das Führungsbauteil 1 schnell und einfach an der vorgesehenen Stelle im Körper des Lebewesens durch Anlage an einer oder mehreren Knochenstrukturen fixieren, beispielsweise anklemmen zu können. Das gezeigte Führungsbauteil 1 ist insbesondere dazu vorgesehen, als interkostales Führungsbauteil 1 zwischen zwei benachbarten bzw. einander gegenüberliegenden Rippen eines Lebewesens angeordnet zu werden. **In** Figur 1 handelt es sich, bezogen auf einen Implantierungszustand des Führungsbauteils 1, um eine dorsale, also dem Rücken des Lebewesens zugewandte Ansicht des Führungsbauteils 1. Zu erkennen sind der im Querschnitt annähernd rechteckförmig ausgebildete Grundkörper 2 sowie ein als schräge Durchgangsbohrung ausgeführter, im Wesentlichen mittig durch den Grundkörper 2 verlaufender Leitungskanal 3. Durch die Schrägbohrung sind besonders wenig bis keine Leitungsumlenkungen zu beobachten, sodass die beabsichtigte Zugentlastung maximiert wird. Überdies findet kein Verhaken und insbesondere kein Knicken der Leitung am Eingang oder Ausgang des Leitungskanals 3 statt, sodass eine Beschädigung der Leitung während der Führung durch das Führungsbauteil 1 verhindert wird. Der Leitungskanal 3 verläuft in der gezeigten Ausführungsform von einer ventralen Oberfläche 8 des Grundkörpers 2 zu einer dorsalen Oberfläche 9 des Grundkörpers 2. Durch den Grundkörper 2 verläuft eine kranial-kaudale Mittelachse 7 des Grundkörpers 2, um die herum der Grundkörper 2 leicht gebogen ist, wie dies insbesondere in Figur 3 erkennbar ist. Der Figur 1 sind des Weiteren einteilig mit dem Grundkörper 2 ausgeformte Befestigungsstrukturen 10 in Form von insgesamt acht Ösen zu entnehmen, die auf der dorsalen Oberfläche 9 des Grundkörpers 2 angeordnet sind und beispielsweise ein Annähen des Führungsbauteils 1 an eine Knochen-, Haut- oder Gewebestruktur ermöglicht. Hierdurch wird bedarfsweise ein zusätzlicher Halt des Führungsbauteils 1 an der vorgesehenen Position im Körper des Lebewesens erreicht. Des Weiteren sind in Figur 1 insgesamt vier Befestigungsöffnungen 11 in dem Grundkörper 2 gezeigt, durch die beispielsweise Fäden oder Schrauben geführt werden können, um das Führungsbauteil 1 durch eine Schraubverbindung zusätzlich an einer Knochen- oder Gewebestruktur des Lebewesens fixieren zu können. Hierdurch wird bedarfsweise ein zusätzlicher Halt des Führungsbauteils 1 an der vorgesehenen Position im Körper des Lebewesens erreicht. Das Führungsbauteil 1 weist an seinem Umfang im Bereich der kaudalen Anlagefläche 4b eine leichte Wölbung auf, die zu einer geringfügigen Übermaßpassung des Führungsbauteils 1 führt und im eingesetzten Zustand des Führungsbauteils 1 die auf die anliegende Knochenstruktur wirkende Anpresskraft erhöht.

Figur 2 zeigt eine perspektivische dorsale Ansicht des Führungsbauteils 1. Die auf der dorsalen Oberfläche 9 des Grundkörpers 2 befindlichen bzw. sichtbaren Bestandteile des Führungsbauteils 1 wurden bereits in Figur 1 erläutert. Zusätzlich ist die Anlagefläche 4 erkennbar, mit der der Grundkörper 2 im implantierten Zustand des Führungsbauteils 1 an einer Knochenstruktur des Lebewesens form- und/oder kraftschlüssig anliegt. Es ist ersichtlich, dass die Anlagefläche 4 als konkave Anlagefläche 4 am Umfang des Führungsbauteils 1 zwischen der dorsalen Oberfläche 9 und der ventralen Oberfläche 8 angeordnet ist. Durch die konkave Form ist das Führungsbauteil 1 insbesondere für konvexe Knochenstrukturen oder Knochenabschnitte wie beispielsweise Rippen geeignet. Bei der Ansicht in Figur 2 handelt es sich bei der Anlagefläche 4 um eine kaudale Anlagefläche 4b des Führungsbauteils 1. Wird das Führungsbauteil 1 beispielsweise zwischen zwei Rippenbögen eingesetzt, liegt die kaudale Anlagefläche 4b an einem kranialen Abschnitt des oberen, dem Kopf zugewandten Rippenbogens an. In der gezeigten Ausführungsform ist die Anlagefläche 4 als am Umfang des Führungsbauteils 1 umlaufende Nut 12 ausgebildet, die Nutseitenwände 13 und einen in den weiteren Figuren gezeigten Nutgrund 14 aufweist. Durch die als Nut 12 ausgebildete Anlagefläche 4 können etwaige Gefäße wie Blutgefäße an der Knochenstruktur quetschfrei aufgenommen werden, sodass die Blutzirkulation an der Knochenstruktur nicht durch das Führungsbauteil 1 beeinträchtigt wird. Dadurch, dass die Nut 12 umlaufend ausgebildet ist, also ohne Unterbrechung durchgehend die Umfangsform des Führungsbauteils 1 festlegt, kann das Führungsbauteil 1 relativ frei an einer Knochenstruktur positioniert werden, beispielsweise in einer leicht angestellten Position oder auch an einer Stelle, an der es von mehreren Knochen umgeben bzw. eingeschlossen wird. Grundsätzlich ist es jedoch genauso denkbar, die Nut 12 nur abschnittsweise auszuführen oder mehrere Nuten 12 vorzusehen.

Figur 3 zeigt eine kaudale Ansicht des zweiteiligen Führungsbauteils 1 im zusammengesteckten Zustand der beiden Führungsbauteilkomponenten 5a, 5b. In dieser Ansicht ist die Anlagefläche 4 erkennbar, mit der der Grundkörper 2 im implantierten Zustand des Führungsbauteils 1 an einer Knochenstruktur des Lebewesens form- und/oder kraftschlüssig anliegt. Es ist ersichtlich, dass die Anlagefläche 4 als konkave Anlagefläche 4 am Umfang des Führungsbauteils 1 zwischen der dorsalen Oberfläche 9 und der ventralen Oberfläche 8 angeordnet ist. Durch die konkave Form ist das Führungsbauteil 1 insbesondere für konvexe Knochenstrukturen oder Knochenabschnitte wie beispielsweise Rippen geeignet. Bei der Ansicht in Figur 3 handelt es sich bei der Anlagefläche 4 um eine kaudale Anlagefläche 4b des Führungsbauteils 1. Wird das Führungsbauteil 1 beispielsweise zwischen zwei Rippenbögen eingesetzt, liegt diese kaudale Anlagefläche 4a an einem kranialen Abschnitt des oberen, dem Kopf zugewandten Rippenbogens an. In der gezeigten Ausführungsform ist die Anlagefläche 4 als am Umfang des Führungsbauteils 1 umlaufende Nut 12 ausgebildet, die Nutseitenwände 13 und einen Nutgrund 14 aufweist. Durch die als Nut 12 ausgebildete Anlagefläche 4 können etwaige Gefäße wie Blutgefäße an der Knochenstruktur quetschfrei aufgenommen werden, sodass die Blutzirkulation an der Knochenstruktur nicht durch das Führungsbauteil 1 beeinträchtigt wird. Dadurch, dass die Nut 12 umlaufend ausgebildet ist, also ohne Unterbrechung durchgehend die Umfangsform des Führungsbauteils 1 festlegt, kann das Führungsbauteil 1 relativ frei an einer Knochenstruktur positioniert werden, beispielsweise in einer leicht angestellten Position oder auch an einer Stelle, an der es von mehreren Knochen umgeben bzw. eingeschlossen wird. Grundsätzlich ist es jedoch genauso denkbar, die Nut 12 nur abschnittsweise auszuführen oder mehrere Nuten 12 vorzusehen. Auf der kaudalen Anlagefläche 4b sind in dem Nutgrund 14 Haltestrukturen 15 in Form von aus dem Nutgrund 14 herausstehenden Noppen ausgebildet, die einteilig mit dem Grundkörper 2 ausgeführt sind. Diese sichern das Führungsbauteil 1 zusätzlich gegen eine Verlagerung gegenüber der Knochenstruktur, indem die Haltestruktur 15 die zwischen der Anlagefläche 4 und der Knochenstruktur wechselwirkende Oberfläche und somit die bestehenden Reibungskräfte erhöht.

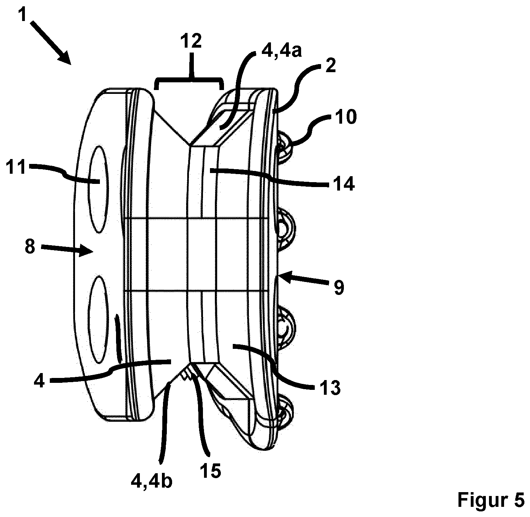

Die Figuren 4 und 5 zeigen jeweils eine seitliche Ansicht des zweiteiligen Führungsbauteils 1, in der nochmals die Ausbildung der Anlagefläche 4 als umlaufende Nut 12 mit Nutseitenwänden 13 und einem Nutgrund 14 ersichtlich wird. Die gezeigte Anlagefläche 4 geht auf der im Bild jeweils unteren Seite in die kaudale Anlagefläche 4b und auf der im Bild jeweils oberen Seite in die kraniale Anlagefläche 4a über.

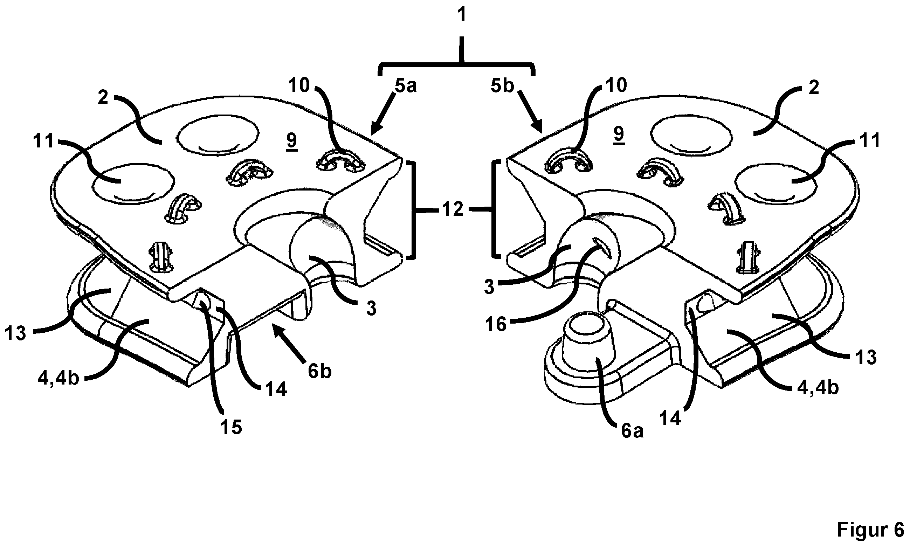

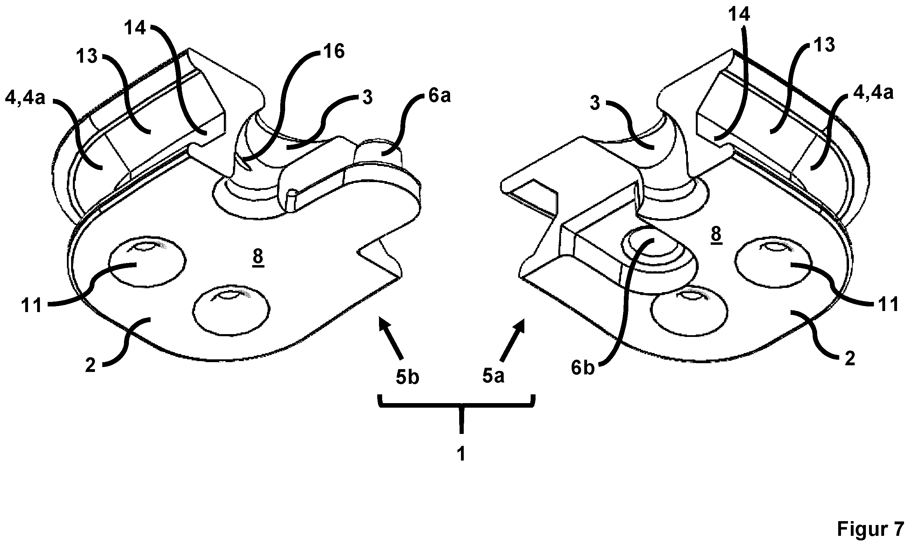

Die Figuren 6 und 7 zeigen nacheinander eine dorsale und eine ventrale Ansicht des zweiteiligen Führungsbauteils 1 mit voneinander getrennten Führungsbauteilkomponenten 5a, 5b. Hierbei ist in beiden Ansichten ein Verbindungsmittel zur Verbindung der beiden Führungsbauteilkomponenten 5a, 5b ersichtlich, nämlich in Form einer Rastnase 6a der Führungsbauteilkomponente 5a und einer korrespondierenden Rastöffnung 6b der Führungsbauteilkomponente 5b. Die Rastnase 6a greift in einem zusammengesetzten Zustand des Führungsbauteils 1 in die Rastöffnung 6b der Führungsbauteilkomponente 5b ein. Durch Zusammenstecken, also Einführen und Verrasten der Rastnase 6a in der Rastöffnung 6b, können die beiden Führungsbauteilkomponenten 5a, 5b zuverlässig, aber lösbar miteinander verbunden werden. Des Weiteren ist erkennbar, dass die Teilungsebene des Führungsbauteils 1 zur Aufteilung in die Führungsbauteilkomponenten 5a, 5b durch den Leitungskanal 3 verläuft. Somit ist ein Teil des Leitungskanals 3 an der einen Führungsbauteilkomponente 5a als Kanal mit halbkreisförmigen Querschnitt angeformt und der andere Teil des Leitungskanals 3 ist an der anderen Führungsbauteilkomponente 5b als Kanal mit halbkreisförmigen Querschnitt angeformt. Durch Zusammensetzen der beiden Führungsbauteilkomponenten 5a, 5b wird der Leitungskanal 3 geschlossen. Auf diese Weise kann eine bereits in dem Körper des Lebewesens verlegte Leitung von dem Leitungskanal 3 des Führungsbauteils 1 umschlossen werden.

In der Wandung des Leitungskanals 3 ist weiterhin eine Fixierungsstruktur 16 angeformt, welche in den Leitungskanal 3 hineinragt. Die Fixierungsstruktur 16 ist beispielsweise durch einen länglichen Materialvorsprung gebildet. Auf diese Weise wird die Leitung innerhalb des Leitungskanals 3 gezielt bzw. bedarfsweise fixiert und zugentlastet. Somit werden unerwünschte Verlagerungen der Leitung reduziert oder verhindert und die Zugstabilität der Leitung vergrößert

Das Führungsbauteil 1 ist bevorzugt aus einem Metall oder Kunststoff gefertigt, der biokompatibel und somit gut verträglich mit den umliegenden Gewebe- und Knochenstrukturen des Lebewesens ist. Das Führungsbauteil 1 kann eine gewisse Grundelastizität aufweisen, um ohne größere Kraftanstrengung an einer Knochenstruktur befestigt, beispielsweise an- oder eingeklemmt werden zu können. Es sollte jedoch auch eine ausreichende Grundstabilität des Führungsbauteils 1 für einen zuverlässigen Halt der Leitung gewährleistet bleiben.

Den in dieser Anmeldung verwendeten anatomischen Richtungsbezeichnungen wie kranial, kaudal, ventral und dorsal soll lediglich ein beispielhafter Charakter zukommen, um die räumliche Vorstellung des Erfindungsgegenstands zu fördern. Keinesfalls sollen die Richtungsbezeichnungen als einschränkend in dem Sinne verstanden werden, dass das erfindungsgemäße Führungsbauteil nur in einer Orientierung und Position in den Körper eines Lebewesens einsetzbar ist. So ist grundsätzlich denkbar, dass beispielsweise die ventrale und dorsale Seite des Führungsbauteils vertauschbar sind, ebenso wie die kraniale und kaudale Seite. Genauso könnte bei einem Schwenken des Führungsbauteils 1 beispielsweise um 90° eine vorherige seitliche Anlagefläche des Führungsbauteils zu einer kranialen oder kaudalen Anlagefläche werden.

## Patentansprüche

1. Intrakorporales Führungsbauteil (1) zum Führen von transkutan verlaufenden Leitungen medizinischer Vorrichtungen innerhalb eines Lebewesens, wobei das Führungsbauteil (1) einen Grundkörper (2) mit einem Leitungskanal (3) zur Aufnahme und Führung der Leitung aufweist, wobei der Grundkörper (2) an seinem Umfang eine oder mehrere Anlageflächen (4, 4a, 4b) aufweist, die zur Anlage an einer Knochenstruktur des Lebewesens ausgebildet sind, wobei der Grundkörper (2) mindestens zwei einander gegenüberliegende Anlageflächen (4, 4a, 4b) aufweist , **dadurch gekennzeichnet, dass** das Führungsbauteil (1) als interkostales Führungsbauteil (1) zum längerfristigen Verbleib ausgebildet ist, dessen Grundkörper (2) eine kraniale Anlagefläche (4a) aufweist, die zur form- und/oder kraftschlüssigen Anlage an einer kranial gelegenen Rippe des Lebewesens vorgesehen ist, und dessen Grundkörper (2) eine kaudale Anlagefläche (4b) aufweist, die zur form- und/oder kraftschlüssigen Anlage an einer kaudal gelegenen Rippe des Lebewesens vorgesehen ist.

2. Intrakorporales Führungsbauteil (1) nach einem der vorhergehenden Ansprüche, **dadurch gekennzeichnet, dass** das Führungsbauteil (1) mehrteilig aus mehreren Führungsbauteilkomponenten (5a, 5b), insbesondere zweiteilig aus zwei Führungsbauteilkomponenten (5a, 5b) gebildet ist.

3. Intrakorporales Führungsbauteil (1) nach Anspruch 2, **dadurch gekennzeichnet, dass** die Führungsbauteilkomponenten (5a, 5b) des mehrteiligen Führungsbauteils (1) Verbindungsmittel zur Herstellung einer insbesondere lösbaren Verbindung untereinander aufweisen.

4. Intrakorporales Führungsbauteil (1) nach einem der vorhergehenden Ansprüche, **dadurch gekennzeichnet, dass** der Grundkörper (2) des Führungsbauteils (1) eine um eine kranial-kaudale Mittelachse (7) des Grundkörpers (2) gebogene Grundform aufweist.

5. Intrakorporales Führungsbauteil (1) nach einem der vorhergehenden Ansprüche, **dadurch gekennzeichnet, dass** der Leitungskanal (3) zwischen einer ventralen Oberfläche (8) des Grundkörpers (2) und einer dorsalen Oberfläche (9) des Grundkörpers (2) verläuft.

6. Intrakorporales Führungsbauteil (1) nach einem der vorhergehenden Ansprüche, **dadurch gekennzeichnet, dass** der Leitungskanal (3) zur Aufnahme und Führung der Leitung als Schrägbohrung durch den Grundkörper (2) ausgeführt ist.

7. Intrakorporales Führungsbauteil (1) nach einem der vorhergehenden Ansprüche, **dadurch gekennzeichnet, dass** der Leitungskanal (3) abschnittsweise eine Querschnittsverengung aufweist und/oder dass eine Fixierungsstruktur des Grundkörpers (2) in den Leitungskanal (3) hineinragt.

8. Intrakorporales Führungsbauteil (1) nach einem der vorhergehenden Ansprüche, **dadurch gekennzeichnet, dass** der Grundkörper (2) mindestens eine einteilig mit dem Grundkörper (2) ausgeformte Befestigungsstruktur (10) aufweist.

9. Intrakorporales Führungsbauteil (1) nach einem der vorhergehenden Ansprüche, **dadurch gekennzeichnet, dass** der Grundkörper (2) mindestens eine Befestigungsöffnung (11) aufweist.

10. Intrakorporales Führungsbauteil (1) nach einem der vorhergehenden Ansprüche, **dadurch gekennzeichnet, dass** die eine oder mehreren Anlageflächen (4, 4a, 4b) als zumindest abschnittsweise am Umfang des Grundkörpers (2) verlaufende Nut (12) oder Nuten (12) ausgebildet sind.

11. Intrakorporales Führungsbauteil (1) nach Anspruch 10, **dadurch gekennzeichnet, dass** am Nutgrund (14) der Nut (12) oder Nuten (12) mindestens eine Haltestruktur (15) angeordnet, insbesondere einteilig mit dem Grundkörper (2) ausgeformt ist.

12. Intrakorporales Führungsbauteil (1) nach einem der vorhergehenden Ansprüche, **dadurch gekennzeichnet, dass** der Durchmesser des Leitungskanals (3) dem Durchmesser der von dem Leitungskanal (3) aufzunehmenden Leitung entspricht.

13. Intrakorporales Führungsbauteil (1) nach einem der vorhergehenden Ansprüche, **dadurch gekennzeichnet, dass** das Führungsbauteil (1) aus einem biokompatiblen Metall oder biokompatiblen Kunststoff gefertigt ist.

## Claims

1. An intracorporeal guide component (1) for guiding transcutaneous line of medical devices within a living organism, wherein the guide component (1) comprises a base body (2) with a line channel (3) for receiving and guiding the line, wherein the base body (2) has, at its periphery, one or more contact surfaces (4, 4a, 4b) on its periphery, which are designed to contact a bone structure of the living organism, wherein the base body (2) comprises at least two opposing contact surfaces (4, 4a, 4b), **characterized in that** the guide component (1) is designed as an intercostal guide component (1) intended for long-term use, whose base body (2) comprises a cranial contact surface (4a) intended for form-fit and/or force-fit contact with a cranially located rib of the living being, and whose base body (2) comprises a caudal contact surface (4b) intended for form-fit and/or force-fit contact with a caudal rib of the living being.

2. An intracorporeal guide component (1) according to any of the preceding claims,
**characterized in that** the guide component (1) is formed in multiple parts from several guide component parts (5a, 5b), in particular in two parts from two guide component parts (5a, 5b).

3. An intracorporeal guide component (1) according to claim 2, **characterized in that** the guide component components (5a, 5b) of the multi-part guide component (1) comprise connecting means for establishing a connection, in particular a releasable connection, between them.

4. An intracorporeal guide component (1) according to any one of the preceding claims, **characterized in that** the base body (2) of the guide component (1) has a basic shape curved around a cranial-caudal central axis (7) of the base body (2).

5. An intracorporeal guide component (1) according to any of the preceding claims,
**characterized in that** the guide channel (3) extends between a ventral surface (8) of the base body (2) and a dorsal surface (9) of the base body (2).

6. An intracorporeal guide component (1) according to any of the preceding claims,
**characterized in that** the line channel (3) for receiving and guiding the guide wire is designed as an inclined bore through the base body (2).

7. An intracorporeal guide component (1) according to any of the preceding claims,
**characterized in that** the line channel (3) has a cross-sectional narrowing in sections and/or that a fixation structure of the base body (2) protrudes into the line channel (3).

8. An intracorporeal guide component (1) according to any of the preceding claims,
**characterized in that** the base body (2) comprises at least one fastening structure (10) formed integrally with the base body (2).

9. An intracorporeal guide component (1) according to any of the preceding claims,
**characterized in that** the base body (2) comprises at least one fastening opening (11).

10. Intracorporeal guide component (1) according to any of the preceding claims,
**characterized in that** the one or more contact surfaces (4, 4a, 4b) are formed as a groove (12) or grooves (12) extending at least in sections along the circumference of the base body (2).

11. Intracorporeal guide component (1) according to claim 10, **characterized in that** at least one retaining structure (15) is arranged at the bottom (14) of the groove (12) or grooves (12), in particular formed integrally with the base body (2).

12. Intracorporeal guide component (1) according to one of the preceding claims,
**characterized in that** the diameter of the line channel (3) corresponds to the diameter of the guide to be received by the line channel (3).

13. Intracorporeal guide component (1) according to one of the preceding claims,
**characterized in that** the guide component (1) is made of a biocompatible metal or biocompatible plastic.

## Revendications

1. Élément de guidage intracorporel (1) destiné à guider des lignes/conduites transcutanées de dispositifs médicaux à l'intérieur d'un être vivant, l'élément de guidage (1) comportant un corps de base (2) muni d'un canal pour ligne/conduite (3) destiné à recevoir et à guider la ligne/conduite, le corps de base (2) présentant sur son pourtour une ou plusieurs surfaces d'appui (4, 4a, 4b) qui sont conçues pour venir en appui contre une structure osseuse de l'être vivant, le corps de base (2) comportant au moins deux surfaces d'appui (4, 4a, 4b) situées à l'opposé l'une de l'autre,
**caractérisé en ce que** l'élément de guidage (1) est conçu comme un élément de guidage intercostal (1) destiné à rester en place à long terme, dont le corps de base (2) présente une surface d'appui crâniale (4a) prévue pour s'appuyer par complémentarité de forme et/ou par adhérence contre une côte de l'être vivant située crânialement, et dont le corps de base (2) présente une surface d'appui caudale (4b) prévue pour s'appuyer par complémentarité de forme et/ou par adhérence contre une côte de l'être vivant située caudalement.

2. Élément de guidage intracorporel (1) selon l'une des revendications précédentes,
**caractérisé en ce que** l'élément de guidage (1) est réalisé en plusieurs parties avec plusieurs composants d'élément de guidage (5a, 5b), en particulier en deux parties avec deux composants d'élément de guidage (5a, 5b).

3. Élément de guidage intracorporel (1) selon la revendication 2,
**caractérisé en ce que** les composants d'élément de guidage (5a, 5b) de l'élément de guidage (1) en plusieurs parties comportent des moyens de liaison permettant d'établir entre eux une liaison notamment amovible.

4. Élément de guidage intracorporel (1) selon l'une des revendications précédentes,
**caractérisé en ce que** le corps de base (2) de l'élément de guidage (1) présente une forme de base courbée autour d'un axe central crânio-caudal (7) du corps de base (2).

5. Élément de guidage intracorporel (1) selon l'une des revendications précédentes,
**caractérisé en ce que** le canal pour ligne/conduite (3) s'étend entre une surface ventrale (8) du corps de base (2) et une surface dorsale (9) du corps de base (2).

6. Élément de guidage intracorporel (1) selon l'une des revendications précédentes,
**caractérisé en ce que** le canal pour ligne/conduite (3) destiné à recevoir et à guider la ligne/conduite est réalisé sous la forme d'un alésage oblique traversant le corps de base (2).

7. Élément de guidage intracorporel (1) selon l'une des revendications précédentes,
**caractérisé en ce que** le canal pour ligne/conduite (3) présente localement un rétrécissement de section transversale et/ou **en ce qu'**une structure de fixation du corps de base (2) fait saillie dans le canal pour ligne/conduite (3).

8. Élément de guidage intracorporel (1) selon l'une des revendications précédentes,
**caractérisé en ce que** le corps de base (2) comporte au moins une structure de fixation (10) formée d'un seul tenant avec le corps de base (2).

9. Élément de guidage intracorporel (1) selon l'une des revendications précédentes,
**caractérisé en ce que** le corps de base (2) comporte au moins une ouverture de fixation (11).

10. Élément de guidage intracorporel (1) selon l'une des revendications précédentes,
**caractérisé en ce que** la ou les surfaces d'appui (4, 4a, 4b) sont réalisées sous forme de rainure (12) ou de rainures (12) s'étendant au moins localement sur le pourtour du corps de base (2).

11. Élément de guidage intracorporel (1) selon la revendication 10,
**caractérisé en ce qu'**au moins une structure de retenue (15) est disposée au fond (14) de la rainure (12) ou des rainures (12) et est notamment formée d'un seul tenant avec le corps de base (2).

12. Élément de guidage intracorporel (1) selon l'une des revendications précédentes,
**caractérisé en ce que** le diamètre du canal pour ligne/conduite (3) correspond au diamètre de la ligne/conduite à recevoir par le canal pour ligne/conduite (3).

13. Élément de guidage intracorporel (1) selon l'une des revendications précédentes,
**caractérisé en ce que** l'élément de guidage (1) est fabriqué à partir d'un métal biocompatible ou d'une matière plastique biocompatible.
